# EUROPEAN PATENT SPECIFICATION

(11) **EP 2 967 751 B1**
(45) Date of publication and mention of the grant of the patent: **26.09.2018**
(21) Application number: 14773432.1
(22) Date of filing: 28.02.2014
(51) Int. Cl.: A61B 18/24, A61B 18/20, A61B 18/22, A61B 18/00

(54) **SMART MULTIPLEXED MEDICAL LASER SYSTEM**
INTELLIGENTES SYSTEM MIT MULTIPLEXIERTEN MEDIZINISCHEN LASERN
SYSTÈME MULTIPLEXÉ INTELLIGENT DE LASER MÉDICAL

(30) Priority: 14.03.2013 US 201313804923; 14.03.2013 US 201313826053
(43) Date of publication of application: 20.01.2016
(73) Proprietor: The Spectranetics Corporation, Colorado Springs, CO 80921-3617 (US)
(72) Inventor: GRACE, Kenneth, P., Woodland Park, CO 80863 (US)
(74) Representative: de Haan, Poul Erik
(86) International application number: PCT/US2014/019283
(87) International publication number: WO 2014/158688

(56) References cited:
- WO-A2-00/57228
- JP-A- 2010 154 978
- US-A- 4 925 265
- US-A- 5 034 010
- US-A- 5 034 010
- US-A- 5 400 428
- US-A1- 2004 057 659
- US-A1- 2006 217 695
- US-B2- 6 539 132

## Description

### BACKGROUND

Laser energy can be transmitted through multiple optical fibers housed in a relatively flexible tubular catheter inserted into a body lumen, such as a blood vessel, ureter, fallopian tube, cerebral artery and the like to remove obstructions in the lumen. Catheters used for laser angioplasty and other procedures can have a central passage or tube which receives a guide wire inserted into the body lumen (e.g., vascular system) prior to catheter introduction. The guide wire facilitates the advancement and placement of the catheter to the selected portion(s) of the body lumen for laser ablation of tissue.

In designing a laser catheter, there are a number of considerations.

For example, laser energy should be controllably and selectively delivered onto single fibers or smaller sub-bundles of subsets of fibers that make up the total number of fibers that are incorporated into the device. This can reduce the overall impact of heat or acoustic shock that can damage tissue adjacent to the treatment site by dividing the transmitted laser energy into smaller packets. It can enable selective treating and targeting of specific zones encountered by the distal tip of the catheter by activating only the fibers required to heat those zones. It can reduce the overall power and energy requirements of the treatment laser by activating smaller portions of fibers in the catheter rather than activating all of the fibers simultaneously. When the instantaneous energy delivered by laser angioplasty catheters is maintained below an "adverse effect" threshold, while at the same time delivering the proper fluence values for tissue ablation, a significant reduction in the incidence of undesirable tissue damage can occur. Previous laser catheters able to energize controllably and selectively fibers used fairly complex scan systems including galvanometer scanners (open and closed loop), piezo type deflection devices, and other mechanical beam or coupler deflection systems. These types of systems typically require a fairly complex and expensive drive and control system. Often, diseased tissue is present in locations where only a portion of the laser catheter's distal tip is in contact with the diseased tissue. It may be desirable to activate only the portion of the distal tip in contact with the diseased tissue. Additionally, laser catheters are used to cut or ablate adhesions holding implanted objects in biological tissue. For safety reasons, it may be desirable to ablate only the tissue in contact with the laser delivery device tip and to avoid ablating tissue adjacent to the wall or artery to prevent perforation.

Notwithstanding these considerations, laser catheters typically energize all of the fibers simultaneously to achieve bulk ablation. Such fiber activation outputs energy from all fibers simultaneously. The resultant instantaneous energy required to achieve the fluence, or energy density values for ablation, can become high enough to induce undesirable tissue damage, including laser induced dissection.

### SUMMARY

These and other needs are addressed by the various aspects, embodiments, and configurations of the present disclosure. The invention is defined in the claims, other embodiments being merely exemplary.

Embodiments include a system, comprising: a laser catheter; and a rotating optical member to receive a laser beam along an optical path and rotate to a selected position to redirect the laser beam from the optical path onto one or more selected optical fibers of a laser catheter, wherein a distal end of the laser catheter irradiates an endovascular structure. The rotating optical member is a wedge prism. The above system further comprises at least one of a detector and proximity sensor to sense: at a first time, a first position of the rotating optical member, wherein at the first position the redirected laser beam irradiates a first, but not a second, set of optical fibers; and at a second time, a second position of the rotating optical member, wherein at the second position the redirected laser beam irradiates the second, but not the first, set of optical fibers. Aspects of the above system further comprise a microprocessor executable controller operable to select, based on one or more of total fiber active area of the laser catheter, imaging information regarding the target and/or non-target endovascular structure(s), target endovascular structure characterization information, and current location and/or orientation of a distal tip of the laser catheter, at least one of a fiber active area for each optical channel, a number of optical channels, a configuration of fibers in an optical channel, an optical channel to be irradiated, and an ordering of optical channel irradiation. Aspects of the above system include wherein the redirected laser beam irradiates plural optical fibers positioned along an arc defined by the laser beam during rotation of the optical member.

The present disclosure can provide a number of advantages depending on the particular aspect, embodiment, and/or configuration. Laser energy can be controllably and selectively delivered onto single fibers or smaller sub-bundles of subsets of fibers that make up the total number of fibers that are incorporated into the device. This can reduce the overall impact of heat or acoustic shock that can damage tissue adjacent to the treatment site by dividing the transmitted laser energy into smaller packets. It can enable selective treating and targeting of specific zones encountered by the distal tip of the catheter by activating only the fibers required to heat those zones. It can reduce the overall power and energy requirements of the treatment laser by activating smaller portions of fibers in the catheter rather than activating all of the fibers simultaneously. Activating portions of the distal tip of the catheter using the treatment laser or other diagnostic light source to probe the area of the lesion contact or tissue disease type and/or location to assess the best method or location of the fibers to be activated at the distal end for treatment. This can be accomplished using a simpler and relatively inexpensive beam deflection mechanism. By using rotating optics and rotational position monitoring using encoders or proximity sensors to fire the laser at the exact time the beam is incident on the target fiber or bundles of fibers, a much simpler multiplexed system can be provided. It can eliminate costly galvanometer scanners and amplifiers with closed loop position feedback electronics. A circular multi-fiber coupler can be fabricated easily. Controllable and selective fiber energizing can enable activating only the portion of the distal tip in contact with the diseased tissue. Additionally, it can ablate only the tissue in contact with the laser delivery device tip and avoid ablating tissue adjacent to the wall or artery to prevent perforation. It can avoid providing too much instantaneous energy and inducing undesirable tissue damage, including laser induced dissection. In other words, it can maintain the instantaneous energy delivered by laser angioplasty catheters below the "adverse effect" threshold, while at the same time delivering the proper fluence values for tissue ablation, thereby providing a significant reduction in the incidence of undesirable tissue damage should occur.

These and other advantages will be apparent from the disclosure.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material".

An "axicon" is an optical element that produces a line image lying along the axis from a point source of light; therefore, it has no definite focal length. An example is a lens with a weak conical surface on one face.

The term "computer-readable medium" as used herein refers to any storage and/or transmission medium that participate in providing instructions to a processor for execution. Such a medium is commonly tangible and non-transient and can take many forms, including but not limited to, non-volatile media, volatile media, and transmission media and includes without limitation random access memory ("RAM"), read only memory ("ROM"), and the like. Non-volatile media includes, for example, NVRAM, or magnetic or optical disks. Volatile media includes dynamic memory, such as main memory. Common forms of computer-readable media include, for example, a floppy disk (including without limitation a Bernoulli cartridge, ZIP drive, and JAZ drive), a flexible disk, hard disk, magnetic tape or cassettes, or any other magnetic medium, magneto-optical medium, a digital video disk (such as CD-ROM), any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored. Computer-readable storage medium commonly excludes transient storage media, particularly electrical, magnetic, electromagnetic, optical, magneto-optical signals.

The terms "determine", "calculate" and "compute," and variations thereof, as used herein, are used interchangeably and include any type of methodology, process, mathematical operation or technique.

A "laser emitter" refers to an end portion of a fiber or an optical component that emits laser light from a distal end of the catheter towards a desired target, which is typically tissue.

A "catheter" is a tube that can be inserted into a body cavity, duct, lumen, or vessel, such as the vasculature system. In most uses, a catheter is a thin, flexible tube ("soft" catheter), though in some uses, it may be a larger, solid less flexible-but possibly still flexible-catheter ("hard" catheter).

"Coronary catheterization" is a generally minimally invasive procedure to access the coronary circulation and/or blood filled chambers of the heart using a catheter. It is performed for both diagnostic and interventional (treatment) purposes.

A "coupler" or "fiber optic coupler" refers to the optical fiber device with one or more input fibers and one or several output fibers. Fiber couplers are commonly special optical fiber devices with one or more input fibers for distributing optical signals into two or more output fibers. Optical energy is passively split into multiple output signals (fibers), each containing light with properties identical to the original except for reduced amplitude. Fiber couplers have input and output configurations defined as M x N. M is the number of input ports (one or more). N is the number of output ports and is always equal to or greater than M. Fibers can be thermally tapered and fused so that their cores come into intimate contact. This can also be done with polarization-maintaining fibers, leading to polarization-maintaining couplers (PM couplers) or splitters. Some couplers use side-polished fibers, providing access to the fiber core. Couplers can also be made from bulk optics, for example in the form of microlenses and beam splitters, which can be coupled to fibers ("fiber pig-tailed").

"Electromagnetic radiation" or "EM radiation" or "EMR" is a form of energy emitted and absorbed by charged particles which exhibits wave-like behavior as it travels through space. EMR has both electric and magnetic field components, which stand in a fixed ratio of intensity to each other, and which oscillate in phase perpendicular to each other and perpendicular to the direction of energy and wave propagation. The electromagnetic spectrum, in order of increasing frequency and decreasing wavelength, consists of radio waves, microwaves, infrared radiation, visible light, ultraviolet radiation, X-rays and gamma rays.

A "lead" is a conductive structure, typically an electrically insulated coiled wire. The electrically conductive material can be any conductive material, with metals and intermetallic alloys common. The outer sheath of insulative material is biocompatible and biostable (e.g., non-dissolving in the body) and generally includes organic materials such as polyurethane and polyimide. Lead types include, by way of non-limiting example, epicardial and endocardial leads. Leads are commonly implanted into a body percutaneously or surgically.

The term "module" as used herein refers to any known hardware, software, firmware, artificial intelligence, fuzzy logic, or combination of hardware and software that is capable of performing the functionality associated with that element.

An "optical fiber" or "laser active fiber" is a flexible, transparent fiber made of an optically transmissive material, such as glass (silica) or plastic, that functions as a waveguide, or "light pipe", to transmit light between the two ends of the fiber.

A "surgical implant" is a medical device manufactured to replace a missing biological structure, support, stimulate, or treat a damaged biological structure, or enhance, stimulate, or treat an existing biological structure. Medical implants are man-made devices, in contrast to a transplant, which is a transplanted biomedical tissue. In some cases implants contain electronics, including, without limitation, artificial pacemaker, defibrillator, electrodes, and cochlear implants. Some implants are bioactive, including, without limitation, subcutaneous drug delivery devices in the form of implantable pills or drug-eluting stents.

### BRIEF DESCRIPTION OF THE DRAWINGS

Fig. 1 depicts a distal tip of a laser catheter containing a lumen;
Fig. 2 depicts a distal tip of a lumenless laser catheter;
Fig. 3 is a cross-sectional view of the distal tip of Fig. 1 approaching target tissue or other endovascular structure;
Fig. 4 is a block diagram of a multiplexed laser catheter according to an embodiment;
Fig. 5 is a block diagram of a multiplexed laser catheter according to an embodiment;
Fig. 6 is a block diagram of a control system according to an embodiment;
Figs. 7A-B depict a fiber array according to an embodiment;
Fig. 8 depicts a multiplexing system according to an embodiment;
Figs. 9A-C depict a fiber array according to an embodiment;
Fig. 10 depicts a control algorithm according to an embodiment;
Fig. 11 depicts a first energizing mode;
Figs. 12A-B depict a second energizing mode;
Figs. 13A-C depict a third energizing mode; and
Figs. 14A-D depict a fourth energizing mode.

### DETAILED DESCRIPTION

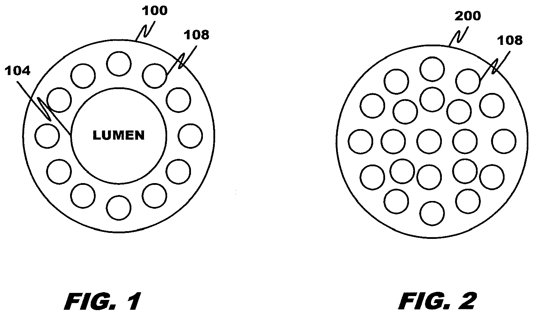

Figs. 1 and 2 depict the working or distal ends of various known prior art laser catheters having plural optical fibers 324 embedded therein. Fig. 1 shows a flexible catheter 100 comprising a catheter lumen 104 to receive an implanted lead or guide wire (not shown) and plural laser emitters 108 positioned around the periphery or diameter of the catheter lumen 104. This type of catheter assembly is sold as a coronary laser atherectomy catheter by the Spectranetics Corporation under the tradenames ELCA™ and Turbo Elite™ (each of which is used for coronary intervention or catheterization such as recanalizing occluded arteries, changing lesion morphology, and facilitating stent placement) and as a laser sheath under the tradename SLSII™ and GlideLight™ (which is used for surgically implanted lead removal). Fig. 2 shows a flexible catheter 200 comprising plural laser emitters 108 packed into the distal end of the catheter. The number of rows of optical fibers and emitters located in the catheter and/or located concentrically around the lumen and the number of optical fibers and emitters in each row can vary by application and are not limited to the depicted configurations. The primary difference between the catheters in Figs. 1 and 2 is the absence of a catheter lumen 104 in the catheter of Fig. 2.

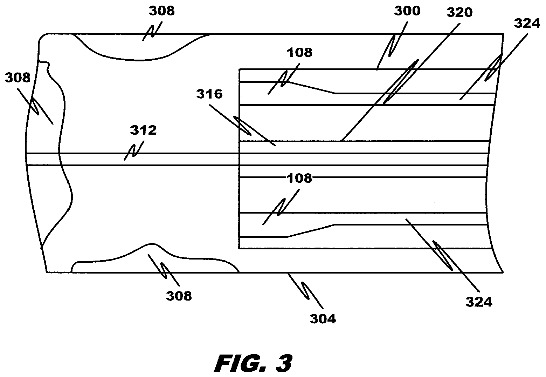

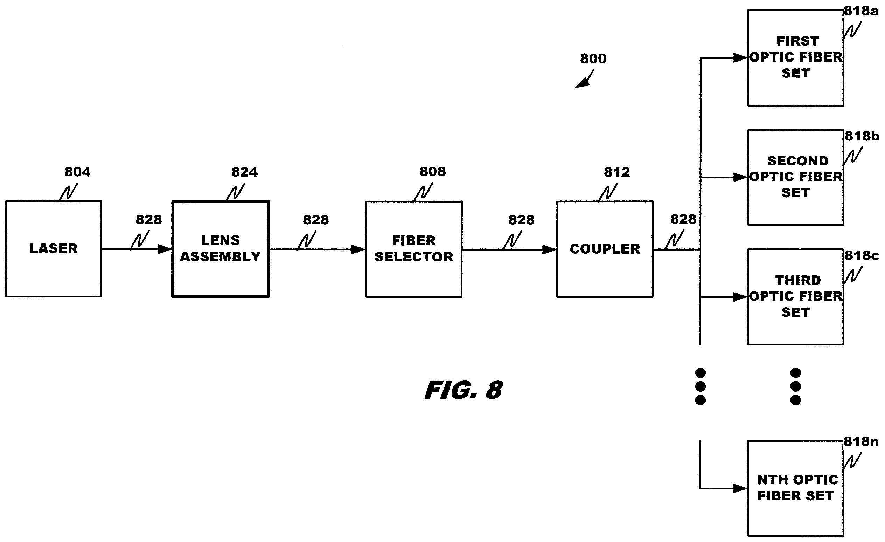

Referring to Fig. 3, a laser ablation catheter 300 is positioned in a body lumen 304, such as a blood vessel, to remove a complete or partial occlusion 308. A guide wire 312 passes through the body lumen 304 and occlusion 308 on the one hand and the catheter lumen 316 formed by a substantially cylindrical inner catheter surface 320 on the other to guide the catheter to the occlusion 308. Laser emitters 108 are positioned at the distal end of the catheter to ablate the occlusion. Optical fiber 324 connects a corresponding emitter to the laser via the proximal end or coupler (Fig. 8).

Fig. 8 depicts a laser assembly 800 including a laser 804, such as a low-temperature excimer laser operating in the ultraviolet spectrum at around 308 nm, a lens assembly 824 (which includes one or more lenses and/or filters), a fiber selector 808 to controllably and selectively energize fibers 108, a coupler 812 to couple to a proximal end of the laser catheter 300, and plural optical channels, each channel being represented by a corresponding first, second, third, ... nth optic fiber set 818a-n. The laser 804 emits laser energy or beam 828, which is directed by the laser assembly 800 to the fiber selector 808. The fiber selector 808 directs the laser energy 828, through the coupler 812, and onto a selected one of the first, second, third,... nth optic fiber set 818a-n. As will be appreciated, the laser 804 can be the same for both treatment and diagnostic laser pulses or different lasers may be employed. When compared to treatment laser pulses, diagnostic laser pulses use lower power.

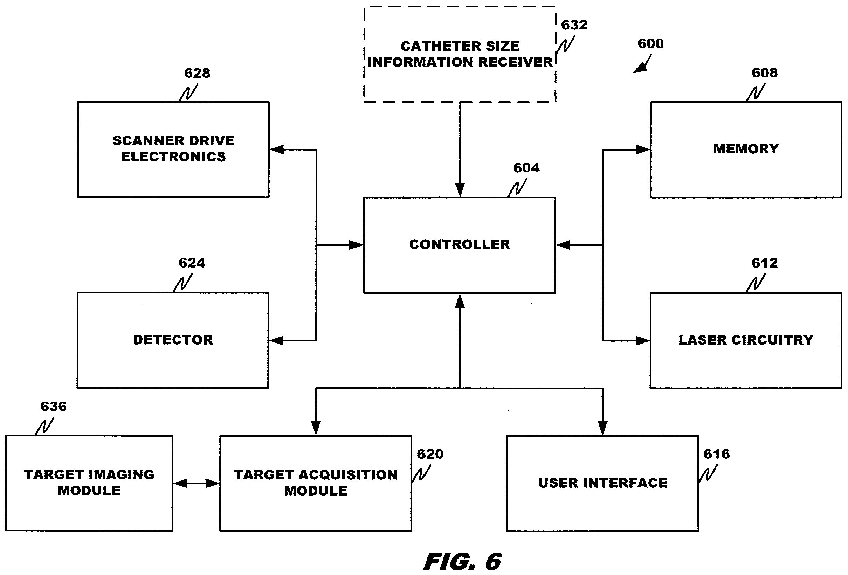

Fig. 6 depicts a control system 600 according to an embodiment. The control system 600 includes a controller 604 (which is typically a microprocessor) in signal and electrical communication with a memory 608, laser circuitry 612 to operate the laser 804, a user interface 616 to receive commands from and provide queries, target tissue or other endovascular structure information, and other feedback to a user, a target acquisition module 620 to determine target tissue or other endovascular structure location and/or characterization information, a detector 624 to select an optical channel from among plural optical channels for energization, scanner drive electronics 628 to operate the fiber selector 808 for energizing selected optical channels, and an optional catheter size information receiver 632 to receive information regarding the total fiber active area at the distal tip of the catheter. The controller 604 can use target tissue or other endovascular structure location and other imaging or diagnostic information, target tissue or other endovascular structure characterization information (such as tissue density, type, location, and configuration) and/or target tissue or other endovascular structure proximity to other non-target tissue structures, such as blood vessel walls, and total fiber active area at the distal end or tip to select not only laser parameters (such as, for each selected optical channel, fluence, energy density value, intensity, an active area, a maximum allowable fluence, a minimum allowable fluence, repetition rate (e.g., lasing on/off time), and lasing train time) but also the number of optical channels, the fiber active area for each optical channel, the fiber configuration for each optical channel, the specific optical channels to energize for target tissue ablation, and the sequence for energizing the selected channels.

The memory 608 can be any computer readable medium and stores, for a current catheterization procedure, a variety of information, including target tissue or other endovascular structure location, characterization information, target tissue or other endovascular structure proximity to non-target tissue structures and other imaging information, laser parameters, optical channel number and configuration, optical channel energizing sequence or ordering, patient information, total fiber active area of the distal end or tip, timestamps, and the like. It can also store various look up tables to enable the controller 604 to configure the optical channels for a currently connected catheter based on the total fiber active area of the catheter. Thus, different models or types or configurations of catheters having different total fiber active areas can have different numbers and configurations of optical channels.

Laser circuitry 612 enables operation of the laser by the controller 604 in response to user commands received by the user interface 616. The laser circuitry 612 is conventional.

The user interface 616 can be any audio, video, and/or tactile interface, such as a keyboard, display, microphone, and the like.

The target acquisition module 620 acquires imaging information via a target imaging (and/or diagnostic) module 636 (hereinafter referred to as target imaging module 636). The imaging information relates not only to the structure of the target tissue or other endovascular structure, such as an occlusion, unwanted tissue growth in proximity to a surgically implanted structure, and the like but also to non-target tissue structures in proximity to the target tissue structure. The target imaging module 636 can be any suitable imaging device, such as, but not limited to, laser induced fluorescence spectroscopy, optical coherence reflectometry, optical coherence tomography, and Raman spectroscopy. The imaging information can be a two, three, or four dimensional representation of the imaged tissue structures.

The detector 624 operatively engages the fiber selector 808 to determine a current optical channel positioned for energizing by the laser and/or a position of a selected optical channel relative to a desired position for the optical channel. The detector 624 can be any suitable configuration, whether a mechanical, optical, electrical, and/or electromagnetic device for tracking movement and/or a current position of the fiber selector 808. It can also be configured as one or more proximity sensors to fire the laser at the precise time that the beam is incident on the target fibers or bundles (or set) of fibers.

The scanner drive electronics 628 one or more of controllably directs the laser beam in a desired orientation and/or controls movement of the fiber selector 808. An example of the former configuration is described in US Patent 5,400,428 to Grace. Grace discloses a dielectric mirror mounted on a galvanometer scanner that is moved to cause successive laser pulses to irradiate different optical channels, thereby enabling each fiber to receive radiation having sufficient fluence while reducing the energy per pulse (or the cw equivalent). Examples of the latter configuration are discussed below.

The catheter size information receiver 632 can be any configuration. For example, it can be based on a lookup table using an identifier of the catheter. The identifier can be provided by a pin sequence or configuration on the proximal end of the catheter. The sequence and/or configuration of pins is mounted to the proximal end of the catheter. The pin arrangement or sequence actuates switches in the catheter's coupler to generate a signal, which is forwarded to the controller 604. Using a lookup table in memory 608 and the signal, the controller 604 can identify the type and/or model of the catheter and therefore the appropriate catheter specifications, requirements, and other operating information. Each type and/or model of catheter has a unique pin sequence to actuate different switches for generating different signals. Other techniques for providing the identifier to the controller 604 may also be employed, such as the techniques discussed in copending US Patent Application Serial No. 13/804,812, entitled "Intelligent Catheter".

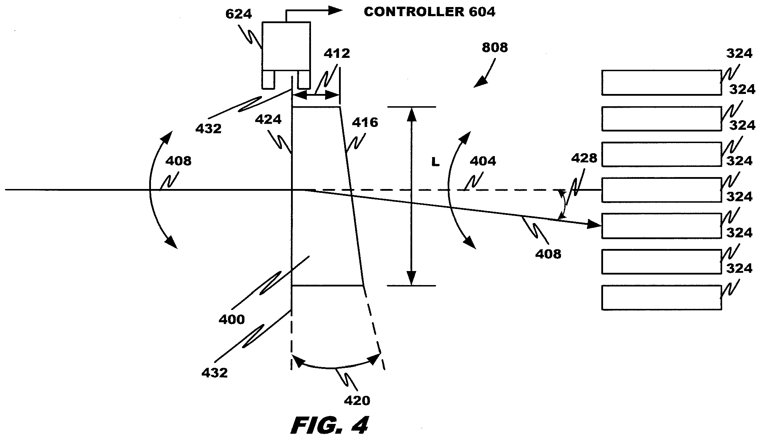

Various configurations of the fiber selector 808 will now be discussed with reference to Figs. 4-5, 7A-B, and 9A-C. Although the fiber selector 808 is shown as being distal to the coupler, it may be positioned within or proximal to the coupler, depending on the application.

Referring to Fig. 4, a first fiber selector configuration is depicted. While Fig. 4 and other figures depict the optical fibers as a linear array for purposes of simplicity, it is to be understood that other fiber arrangements can be employed, particularly fibers oriented in a three dimensional array.

The fiber selector 808 comprises a rotating wedge optical member 400 positioned in the optical path 404 of the laser beam 408 to redirect, by optical refraction, the laser beam onto one or more selected fibers 324, with the optical fiber(s) irradiated at any one time corresponding to an optical channel and optical fiber(s) irradiated at different times corresponding to different optical channels. As will be appreciated, a wedge prism can be uncoated or coated with an anti-reflection coating and can deviate an angle of an incident beam. Typically, the wedge prism deviates the angle of the laser beam by an amount ranging from about 70 to about 20 degrees. The wedge optical member 400 typically has a thickness that is dependent upon the desired beam deviation, which is also a function of the size of the coupler. This causes the laser beam 408 to be diverted at an angle Ω 428 relative to the optical path 404 along a diverging optical path. The motor-driven rotation of the optical member can be at fixed and/or variable speeds.

Alternatively, plural wedge optical members can be used to redirect the laser beam. For example, two wedge prisms can be used as an anamorphic pair to steer the beam anywhere within a circle described by the full angle 4θ, where θ is the deviation from a single prism. This beam steering is accomplished by rotating the two wedge prisms independently of each other.

The position of the wedge optical member 400 can be determined by the detector 624 based upon, for example, radiation reflected by a locating member 432, which rotates simultaneously and in an amount related to rotation of the wedge optical member 400. For example, the locating member 432 can be encoded with encoding elements that reflect light uniquely or substantially uniquely for any position around the circumference of the locating member 432. To produce the unique light reflectance can be the result of the encoding elements being differently sized, spaced, and/or colored. An example of such encoding elements is a bar code. The detector 624 emits light onto the locating member, detects the reflected spectra, and maps the reflected spectra against a lookup table that indexes each absolute and/or relative position around the locating member 432 against a corresponding set of reflected spectra. Based on the comparison, a locating signal is generated and sent to the controller 604, which then instructs a subcontroller (not shown), which further instructs the motor (not shown) to rotate the wedge optical member 400 a selected angle to align the selected optical channel with the redirected laser beam 408. By incorporating the optical encoder, the laser can be fired at the time the beam is deflected to the position to couple into the desired fiber or bundle of fibers. While tracking the position of the wedge optical member 400 is discussed with reference to an optical encoder, other types of encoders may be employed, such as mechanical, electrical, and/or electromagnetic position tracking devices.

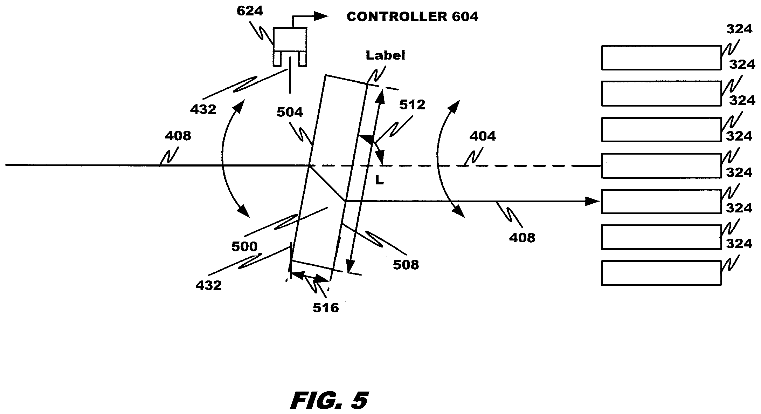

Referring to Fig. 5, a second fiber selector configuration is depicted.

The fiber selector 808 comprises a rotating parallel-faced optical member 500 positioned in the optical path 404 of the laser beam 408 to redirect, by optical refraction, the laser beam onto one or more selected fibers 324. The parallel-faced optical member 500 comprises opposing parallel faces 504 and 508 and is inclined relative to the optical path 404 at an angle δ 512 sufficient to deviate the laser beam 408 from the optical path 408 by an angle ranging from about 70 to about 20 degrees. The optical member 500 typically has a thickness that is dependent upon the desired beam deviation, which is a also a function of the size of the coupler. The inclined parallel surfaces 504 and 512 cause the laser beam 408 to be diverted and offset relative to and substantially parallel to the optical path 404.

In the first and second fiber selector configurations, the direction of rotation, whether clockwise, counterclockwise or both, is a matter of design choice.

In each of the first and second fiber selector configurations, complete rotation of the optical member causes the laser beam to trace, or define, a circle on the optical fibers. Partial rotation of the optical member causes the laser beam to trace a partial circle, with the length of the arc being proportional to the degree of rotation of the optical member. Any optical fiber positioned along the arc is irradiated by the incident redirected laser beam.

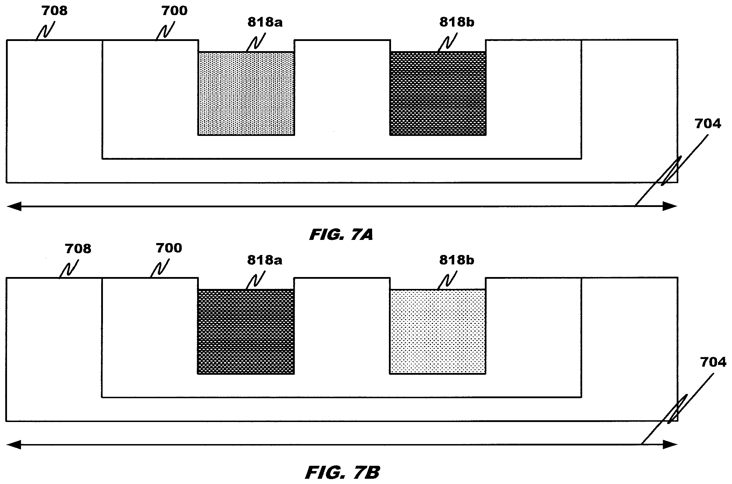

Referring to Figs. 7A-B, a third fiber selector configuration is depicted. This configuration is further discussed in US Patent 5,400,428 mentioned above.

A grooved fiber holder 700 holds two equally sized bundles of fibers 818a,b, each bundle corresponding to a different optical channel. Bundles 818a,b are centered upon the same linear transverse axis 704. The laser beam is focused so that the first incident beam pulse irradiates all of fiber bundle 818b (Fig. 7A), which is half of the total fibers. Either the beam or the fibers are then shifted so that next pulse of the laser beam is focused on bundle 818a (Fig. 7B). The grooved fiber holder 700 can be translated along the linear transverse axis 704 by displacing the holder 700 laterally in a carrier member 708, such as energizing a piezoelectric stack or a motor.

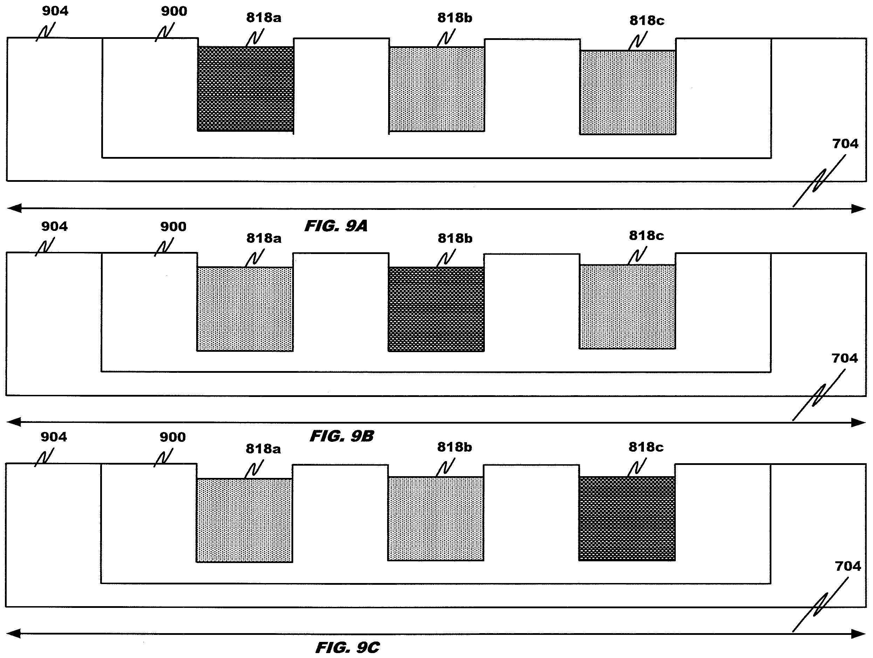

Referring to Figs. 9A-C, a fourth fiber selector configuration is depicted. Figs. 9A-C illustrate three bundles of optical fibers 818a, 818b and 818c, each bundle corresponding to a different optical channel. Each bundle of fibers 818a, 818b and 818c contains 1/3 of the total number of fibers. The optical fiber bundles are disposed in a grooved fiber holder 900 that moves laterally within a holder 904 as discussed above. First scan position (Fig. 9A) irradiates fiber bundle 818a. In a second scan position (Fig. 9B), the beam and fibers are moved relative to one another to irradiate the second fiber bundle 818b. In a third scan position (Fig. 9C), the next laser pulse is directed at the third bundle of optical fibers 818c. As in FIG. 5a, the fiber bundles are disposed in a linear manner along the same transverse axis 320 to provide for linear scanning.

As will be appreciated, other fiber selector configurations may be employed.

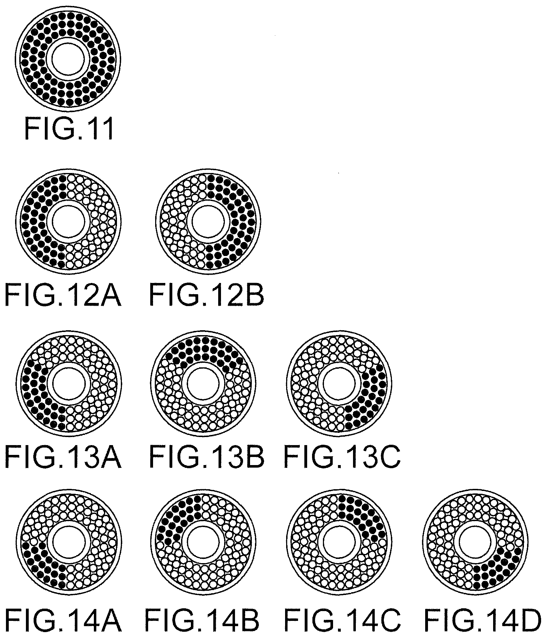

Regardless of the fiber selector configuration employed, the distal end or tip of the catheter energizes all or part of the laser emitters 108 as shown in Figs. 11, 12A-B, 13A-C, and 14A-D. As noted, the portion of the laser emitters 108 energized, or the number of optical channels employed, depend on one or more of target tissue or other endovascular structure location, target tissue or other endovascular structure characterization information (such as tissue density, type, location, and configuration), current location and/or orientation of the distal tip of the catheter, and/or target tissue or other endovascular structure proximity to other non-target tissue structures, and total fiber active area at the distal end or tip. In Figs. 11, 12A-B, 13A-C, and 14A-D, darkened laser emitters refer to those being energized while undarkened laser emitters refer to those not being energized.

Fig. 11 depicts a first operating mode in which all of the laser emitters 108 in the distal tip of the catheter are energized simultaneously.

Figs. 12A-B depict a second operating mode for a 2-way multiplexing configuration in which one-half of the laser emitters 108 are energized simultaneously at a first time and the other half of the laser emitters 108 are energized simultaneously at a second (different) time. This operating mode can be produced by any of the first, second, and third fiber selector configurations.

Figs. 13A-C depict a third operating mode for a 3-way multiplexing configuration in which a first one-third of the laser emitters 108 is energized simultaneously at a first time, a second one-third of the laser emitters 108 is energized simultaneously at a second time, and a third one-third of the laser emitters 108 is energized simultaneously at a third time. The first, second, and third times are different. This operating mode can be produced by any of the first, second, and fourth fiber selector configurations.

Figs. 14A-D depict a fourth operating mode for a 4-way multiplexing configuration in which a first one-quarter of the laser emitters 108 is energized simultaneously at a first time, a second one-quarter of the laser emitters 108 is energized simultaneously at a second time, a third one-quarter of the laser emitters 108 is energized simultaneously at a third time, and a fourth one-quarter of the laser emitters 108 is energized simultaneously at a fourth time. The first, second, third, and fourth times are different. This operating mode can be produced by any of the first and second fiber selector configurations.

As will be appreciated, multiplexing may be performed up to N ways, with N being a whole number. Multiplexing is not limited to 2, 3, and 4 ways as shown in the above figures.

As will be further appreciated, the geometrical pattern of laser emitters 108 energized can be different from those shown. The laser emitters 108 can be energized along an arc, be randomly distributed, and/or be uniformly or nonuniformly distributed around the circumference of the distal tip of the catheter.

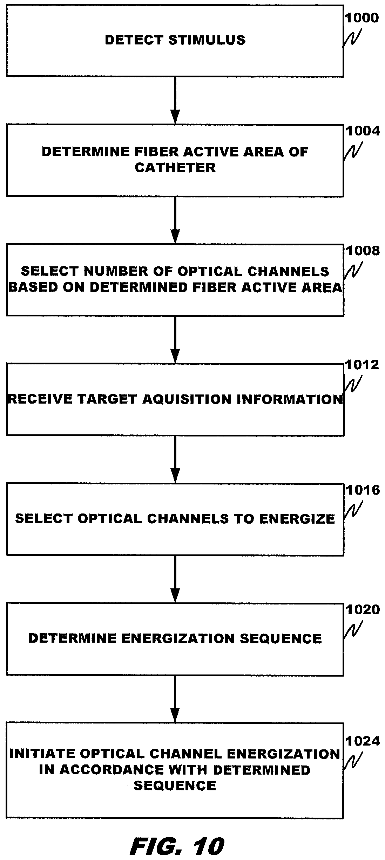

An operation of the controller 604 will now be described with reference to Fig. 10.

The operation commences in step 1000 in which the controller 604 detects a stimulus. The stimulus can be, for example, a command received from an operator, such as a physician, via the user interface 616.

The controller 604 determines a total fiber active area of the catheter currently coupled to the coupler 812.

The controller 604, in step 1008, selects a number of optical channels based on the determined total fiber active area. As noted, this can be done using a lookup table. In other configurations, the controller 604 can use other information, particularly imaging information (including target tissue or other endovascular structure location, target tissue or other endovascular structure characterization information (such as tissue density, type, location, and configuration), current location and/or orientation of the distal tip of the catheter, and/or target tissue or other endovascular structure proximity to other non-target tissue structures) received from the target acquisition module 620 in addition to or lieu of the total fiber active area in selecting the number of optical channels. In that event, step 1008 would follow step 1012.

In one application, the controller 604 selects one or more of a fiber active area for each optical channel, a number of optical channels, a configuration of fibers in an optical channel, an optical channel to be irradiated, and an ordering of optical channel irradiation based on one or more of total fiber active area of the laser catheter, imaging information regarding the target and/or non-target endovascular structure(s), target endovascular structure characterization information, and current location and/or orientation of a distal tip of the laser catheter.

In step 1012, the controller 604 receives target acquisition information from the target acquisition module 620 and indirectly from the target imaging module 636. The target acquisition information is typically imaging information. In some configurations, additional wavelengths of light are launched down the fibers and through the laser emitters in the catheter and returned or reflected light analyzed to determine reflectivity and absorption data. This can be used to determine target and/or non-target tissue or other endovascular structure location, type, and/or contact area (area of contact between the distal tip and structure of the target tissue or other endovascular structure).

In step 1016, the controller 604 selects which of the optical channels to energize to ablate the target tissue or other endovascular structure. This can be determined based upon any or all of the information referenced in the prior paragraph and/or user input.

In step 1020, the controller 604 determines an energization sequence. The sequence governs which optical channels are energized and in what order and times. This can be determined based upon any or all of the information referenced previously including imaging information and user input.

In step 1024, the controller 604 initiates optical channel energization in accordance with the determined energization sequence.

Furthermore, while the exemplary aspects, embodiments, and/or configurations illustrated herein show the various components of the system collocated, certain components of the system can be located remotely, at distant portions of a distributed network, such as a LAN and/or the Internet, or within a dedicated system. Thus, it should be appreciated, that the components of the system can be combined in to one or more devices, such as a base unit, or collocated on a particular node of a distributed network.

## Claims

1. An system, comprising:
a laser catheter (100); and
a rotating wedge prism (400) configured to receive a laser beam along an optical path and rotate to a selected position to redirect, by optical refraction, the laser beam (408) from the optical path onto one or more selected sets (818) of optical fibers (324) of a laser catheter, wherein a distal end of the laser catheter irradiates an endovascular structure;
at least one of a detector (624) and proximity sensor configured to sense:
at a first time, a first position of the rotating wedge prism, wherein at the first position, the redirected laser beam (408) irradiates a first (818a), but not a second (818b), set of optical fibers; and
at a second time, a second position of the rotating wedge prism,
wherein at the second position, the redirected laser beam (408) irradiates the second (818b), but not the first (818a), set of optical fibers.

2. The system of claim 1, further comprising a second rotating wedge prism, wherein the two wedge prisms are adapted to rotate independently of each other.

3. The system of claim 1, further comprising:
a microprocessor executable controller (604) operable to select one or more optical channels, wherein each optical channel corresponds to one or more selected sets of optical fibers for each optical channel.

4. The system of claim 3, wherein the microprocessor executable controller (604) is operable to select at least one of a number optical channels, a fiber active area for each optical channel, a configuration of fibers in an optical channel, an optical channel to be irradiated, and an ordering of optical channel irradiation.

5. The system of claim 4, wherein microprocessor executable controller (604) is operable to select one or more optical channels based on one or more of total fiber active area of the laser catheter, imaging information regarding the target and/or non-target endovascular structure(s), target endovascular structure characterization information, and current location and/or orientation of a distal tip of the laser catheter.

6. The system of claim 1, wherein the redirected laser beam (408) irradiates plural optical fibers (324) positioned along an arc defined by the laser beam during rotation of the wedge prism (400).

7. The system of claim 4, wherein the microprocessor executable controller (604) is configured to select the number of optical channels and fiber active area per optical channel to maintain a level of energy delivered by the irradiated optical channel below an adverse effect threshold but above a level to provide a proper fluence value for tissue ablation.

8. The system of claim 4, wherein the microprocessor executable controller (604) is configured to select a first number of optical channels for a first catheter and a second number of optical channels for a second catheter, wherein the first and second numbers of optical channels are different, and wherein a first total fiber active area of the first catheter is different from a second total fiber active area of the second catheter.

9. The system of claim 4, wherein the microprocessor executable controller (6 04) is configured to select the at least one set of optical fibers for each optical channel, a number of optical channels, a configuration of fibers in an optical channel, an optical channel to be irradiated, and an ordering of optical channel irradiation based on total fiber active area of the laser catheter.

10. The system of claim 4, wherein the microprocessor executable controller (604) further selects one or more optical channels based on an area of contact of the distal tip with the target and/or non-target endovascular structure.

11. The system of claim 1, further comprising:
the rotating wedge prism (400) adapted to receive a laser beam (408) along an optical path and rotate to a selected position to redirect the laser beam from the optical path onto one or more selected optical fibers of the laser catheter, wherein the distal end of the laser catheter irradiates the target endovascular structure.

## Patentansprüche

1. System, umfassend:
einer Laserkatheter (100) sowie
ein rotierendes Keilprisma (400), das so konfiguriert ist, dass es einen Laserstrahl entlang einem optischen Weg empfängt und sich in eine ausgewählte Position dreht, um den Laserstrahl (408) durch optische Refraktion von dem optischen Weg auf einen oder mehrere ausgewählte Sätze (818) optischer Fasern (324) eines Laserkatheters zurückzuleiten, wobei ein distales Ende des Laserkatheters eine endovaskuläre Struktur bestrahlt;
zumindest einen Detektor (624) oder Proximitätssensor, der so konfiguriert ist, dass er abtastet:
zu einem ersten Zeitpunkt, eine erste Position des rotierenden Keilprismas, wobei in der ersten Position der zurückgeleitete Laserstrahl (408) einen ersten (818a), nicht jedoch einen zweiten (818b) Satz optischer Fasern bestrahlt; und
zu einem zweiten Zeitpunkt, eine zweite Position des rotierenden Keilprismas, wobei in der zweiten Position der zurückgeleitete Laserstrahl (408) den zweiten (818b), nicht jedoch den ersten (818a) Satz optischer Fasern bestrahlt.

2. System nach Anspruch 1, weiterhin umfassend ein zweites rotierendes Keilprisma, wobei die zwei Keilprismen so eingerichtet sind, dass sie unabhängig voneinander rotieren.

3. System nach Anspruch 1, weiterhin umfassend:
einen durch Mikroprozessor ausführbaren Controller (604), der so arbeitet, dass er einen oder mehrere optische Kanäle auswählt, wobei jeder optische Kanal einem oder mehreren ausgewählten Sätzen optischer Fasern für jeden optischen Kanal entspricht.

4. System nach Anspruch 3, wobei der durch Mikroprozessor ausführbare Controller (604) so arbeitet, dass er mindestens einen einer Anzahl optischer Kanäle, einen faseraktiven Bereich für jeden optischen Kanal, eine Konfiguration von Fasern in einem optischen Kanal, einen zu bestrahlenden, optischen Kanal sowie eine Reihenfolge der Bestrahlung optischer Kanäle auswählt.

5. System nach Anspruch 4, wobei der durch Mikroprozessor ausführbare Controller (604) so arbeitet, dass er einen oder mehrere optische Kanäle auf der Basis eines(einer) oder mehrerer der folgenden auswählt: eines gesamten faseraktiven Bereichs des Laserkatheters, Bildgebungsinformationen in Bezug auf die endovaskuläre(n) Ziel- und/oder Nicht-Zielstruktur(en), endovaskuläre Zielstruktur-Charakterisierungsinformationen sowie aktuelle Position und/oder Ausrichtung einer distalen Spitze des Laserkatheters.

6. System nach Anspruch 1, wobei der zurückgeleitete Laserstrahl (408) mehrfache optische Fasern (324) bestrahlt, die entlang einem durch den Laserstrahl bei Rotation des Keilprismas (400) definierten Bogen positioniert sind.

7. System nach Anspruch 4, wobei der durch Mikroprozessor ausführbare Controller (604) so konfiguriert ist, dass er die Anzahl optischer Kanäle und den faseraktiven Bereich pro optischen Kanal so auswählt, dass ein durch den bestrahlten, optischen Kanal abgegebener Energielevel unterhalb eines Schwellenwertes mit nachteiligen Auswirkungen, jedoch oberhalb eines Levels, um einen richtigen Fluenzwert zur Gewebeablation vorzusehen, gehalten wird.

8. System nach Anspruch 4, wobei der durch Mikroprozessor ausführbare Controller (604) so konfiguriert ist, dass er eine erste Anzahl von optischen Kanälen für einen ersten Katheter und eine zweite Anzahl von optischen Kanälen für einen zweiten Katheter auswählt, wobei die erste und zweite Anzahl von optischen Kanälen verschieden sind, und wobei ein erster gesamter faseraktiver Bereich des ersten Katheters von einem zweiten gesamten faseraktiven Bereich des zweiten Katheters verschieden ist.

9. System nach Anspruch 4, wobei der durch Mikroprozessor ausführbare Controller (604) so konfiguriert ist, dass er den mindestens einen Satz optischer Fasern für jeden optischen Kanal, eine Anzahl optischer Kanäle, eine Konfiguration von Fasern in einem optischen Kanal, einen zu bestrahlenden, optischen Kanal sowie eine Reihenfolge von Bestrahlungen optischer Kanäle auf der Basis des gesamten faseraktiven Bereichs des Laserkatheters auswählt.

10. System nach Anspruch 4, wobei der durch Mikroprozessor ausführbare Controller (604) weiterhin einen oder mehrere optische Kanäle aufgrund einer Kontaktfläche der distalen Spitze mit der endovaskulären Ziel- und/oder Nicht-Zielstruktur auswählt.

11. System nach Anspruch 1, weiterhin umfassend:
das rotierende Keilprisma (400), das so eingerichtet ist, dass es einen Laserstrahl (408) entlang einem optischen Weg empfängt und sich in eine ausgewählte Position dreht, um den Laserstrahl von dem optischen Weg auf eine oder mehrere ausgewählte optische Fasern des Laserkatheters zurückzuleiten, wobei das distale Ende des Laserkatheters die endovaskuläre Zielstruktur bestrahlt.

## Revendications

1. Système, comprenant :
un cathéter à laser (100) ; et
un prisme rotatif en coin (400) configuré pour recevoir un faisceau laser le long d'un chemin optique et pour tourne jusqu'à une position sélectionnée pour rediriger, par réfraction optique, le faisceau laser (408) depuis le chemin optique sur un ou plusieurs ensembles sélectionnés (818) de fibres optiques (324) d'un cathéter à laser, dans lequel une extrémité distale du cathéter à laser irradie une structure endovasculaire ;
au moins l'un d'un détecteur (624) et d'un capteur de proximité configuré pour capter :
à un premier instant, une première position du prisme rotatif en coin, dans lequel à la première position, le faisceau laser redirigé (408) irradie un premier (818a), mais pas un second (818b), ensemble de fibres optiques ; et
à un second instant, une seconde position du prisme rotatif en coin, dans lequel à la seconde position, le faisceau laser redirigé (408) irradie le second (818b), mais pas le premier (818a), ensemble de fibres optiques.

2. Système selon la revendication 1, comprenant en outre un second prisme rotatif en coin, dans lequel les deux prismes en coin sont conçus pour tourner indépendamment l'un de l'autre.

3. Système selon la revendication 1, comprenant en outre :
une unité de commande exécutable par microprocesseur (604) pouvant être mise en oeuvre pour sélectionner un ou plusieurs canaux optiques, dans lequel chaque canal optique correspond à un ou plusieurs ensembles sélectionnés de fibres optiques pour chaque canal optique.

4. Système selon la revendication 3, dans lequel l'unité de commande exécutable par microprocesseur (604) peut être mise en oeuvre pour sélectionner au moins l'un d'un certain nombre de canaux optiques, d'une zone active de fibres pour chaque canal optique, d'une configuration de fibres dans un canal optique, d'un canal optique à irradier et d'un ordonnancement d'irradiation de canal optique.

5. Système selon la revendication 4, dans lequel l'unité de commande exécutable par microprocesseur (604) peut être mise en oeuvre pour sélectionner un ou plusieurs canaux optiques sur la base d'une ou plusieurs d'une zone active totale de fibres du cathéter à laser, d'informations d'imagerie quant à la ou aux structure(s) endovasculaire(s) ciblée(s) et/ou non ciblée(s), d'informations de caractérisation de structure endovasculaire ciblée, et d'un emplacement courant et/ou orientation d'un embout distal du cathéter à laser.

6. Système selon la revendication 1, dans lequel le faisceau laser redirigé (408) irradie plusieurs fibres optiques (324) positionnées le long d'un arc défini par le faisceau laser pendant la rotation du prisme en coin (400).

7. Système selon la revendication 4, dans lequel l'unité de commande exécutable par microprocesseur (604) est configurée pour sélectionner le nombre de canaux optiques et la zone active de fibres par canal optique pour maintenir un niveau d'énergie délivrée par le canal optique irradié au-dessous d'un seuil d'effet nuisible, mais au-dessus d'un niveau pour fournir une valeur de fluence appropriée pour l'ablation de tissu.

8. Système selon la revendication 4, dans lequel l'unité de commande exécutable par microprocesseur (604) est configurée pour sélectionner un premier nombre de canaux optiques pour un premier cathéter et un second nombre de canaux optiques pour un second cathéter, dans lequel les premier et second nombres de canaux optiques sont différents et dans lequel une première zone active totale de fibres du premier cathéter diffère d'une seconde zone active totale de fibres du second cathéter.

9. Système selon la revendication 4, dans lequel l'unité de commande exécutable par microprocesseur (604) est configurée pour sélectionner l'au moins un ensemble de fibres optiques pour chaque canal optique, un certain nombre de canaux optiques, une configuration de fibres dans un canal optique, un canal optique à irradier et un ordonnancement d'irradiation de canal optique sur la base de la zone active totale de fibres du cathéter à laser.

10. Système selon la revendication 4, dans lequel l'unité de commande exécutable par microprocesseur (604) sélectionne en outre un ou plusieurs canaux optiques sur la base d'une zone de contact de l'embout distale avec la structure endovasculaire ciblée et/ou non ciblée.

11. Système selon la revendication 1, comprenant en outre :
le prisme rotatif en coin (400) conçu pour recevoir un faisceau laser (408) le long d'un chemin optique et tourner jusqu'à une position sélectionnée pour rediriger le faisceau laser depuis le chemin optique sur une ou plusieurs fibres optiques sélectionnées du cathéter à laser, dans lequel l'extrémité distale du cathéter à laser irradie la structure endovasculaire ciblée.
